# EUROPEAN PATENT SPECIFICATION

(11) **EP 3 248 633 B1**
(45) Date of publication and mention of the grant of the patent: **31.10.2018**
(21) Application number: 17171375.3
(22) Date of filing: 16.05.2017
(51) Int. Cl.: A61M 5/14, A61M 5/148, A61M 5/152

(54) **INFUSION DEVICE OF A MEDICAL SOLUTION**
INFUSIONSGERÄT FÜR MEDIZINISCHE LÖSUNG
DISPOSITIF DE PERFUSION POUR SOLUTION MÉDICALE

(30) Priority: 26.05.2016 IT UA20163823
(43) Date of publication of application: 29.11.2017
(73) Proprietor: ADRIA.MED. S.R.L., 65028 Tocco da Casauria (Pescara) (IT)
(72) Inventor: Di Florio, Francesco, 65028 Tocco da Casauria (Pescara) (IT); Di Florio, Armando, 65028 Tocco da Casauria (Pescara) (IT)
(74) Representative: Carangelo, Pierluigi

(56) References cited:
- EP-A1- 0 172 586
- EP-A1- 0 464 761
- WO-A1-2004/002555
- US-A- 4 904 239
- US-A- 5 897 530
- US-A1- 2006 229 558

## Description

The present invention relates to an infusion device of medical solutions containing drugs or other physiologically useful substances.

Portable devices for infusion of medical solutions are widely used. They consist of an elastomeric pump, i.e. a device that allows the continuous dispensing of a medical solution at a flow rate substantially constant and predetermined.

The first infusion pumps were electrical and functioned with a microchip and software that established the infusion rate thereof. They were very large devices, weighing about 1 kg, which limited portability. As a result, the patient's therapy was to be carried out in hospitalization.

With the advent of elastomeric pumps, a real revolution in the field has occurred. This technology has made it possible to achieve outstanding results in terms of precision and constancy of infusion at noticeably lower costs than electronic pumps. The possibility of using the device outside the health facilities has enabled a considerable reduction in the costs of hospitalization.

The elastomeric pump consists of a tank that is shaped like a balloon made of elastomeric material designed to ideally exert a more or less constant pressure on the fluid contained therein. Such a fluid is pushed along an infusion line directly into a vein, subcutaneously, around a plexus, into a joint or epidurally, depending on the needs.

The solution in the tank reaches the patient through said infusion line at the distal end (i.e. the patient side) of which there is a flow restrictor that according to its own section/length determines the hourly flow rate of the system. According to Hagen-Poiseuille's law, the resistance is proportional to the length of the infusion line and inversely proportional to the fourth power of the radius of the infusion section.

Therefore, the hourly flow rate of the elastomeric system is a combination of the following factors:
- pressure exerted by the elastomeric balloon
- flow restrictor inner diameter
- flow restrictor length (impedance)
- viscosity of the solution to be infused.

Elastomeric pumps are mechanically actuated and operated, easy to prepare, do not require programming as the flow is preset, do not need batteries and to be connected to a power source, are reliable and have high security standards. However, although it is desirable to ensure a steady flow of solution dispensed, it has been seen that in fact at the beginning of the infusion and at its end, an increase occurs in the dispensing speed that results in a release of an initial and of a final bolus of solution to the patient. This phenomenon is due to the fact that the elastomeric pump cannot apply a substantially constant pressure throughout the treatment period.

The pressure exerted by the balloon depends in part on the particular chemical-physical and elastic properties of the materials used as elastomer. Silicone elastomer or polyisoprene is normally used. The materials used to date have a good shape memory and tend to return to their starting condition, i.e. the condition in which they are before being filled with the liquid solution.
An infusion device according to the preamble of claim 1 is known from document US 4 904 239 A.

It was however seen by the inventors of the present invention that by suitably shaping the elastomeric pump, greater uniformity of the dispensing can be achieved, in particular minimizing the dispensing speed peaks at the beginning and at the end of the treatment.

An object of the invention is to provide a device for the infusion of a medical solution that allows the dispensing of the solution with a flow as constant as possible over time.

This object is achieved by an infusion device as outlined in the accompanying claims, whose definitions are an integral part of the present description.

Further features and advantages of the present invention will appear more clearly from the description of some preferred embodiments thereof, made hereinafter by way of a non-limiting example only, with reference to the following figures, in which:
Figure 1 shows a lateral sectional view of the elastomeric pump according to the invention;
Figure 2 shows a lateral sectional exploded view of the elastomeric pump in figure 1;
Figures 3A and 3B show a top view of the infusion device of the invention with empty and full solution tank, respectively;
Figure 4 shows a perspective view of the device in figure 4B inserted in a rigid casing.

With reference to the figures, the infusion device of a medical solution, shown as a whole with reference numeral 1, comprises a pumping unit 2 connected to an infusion line 3.

The pumping unit 2 comprises an outer casing 4 containing an elastomeric pump 5.

Casing 4 is made of a flexible transparent or semi-transparent plastic material. For example, it may be made from polyvinyl chloride. This solution allows limiting the size of the device, lightening the structure thereof and making its construction particularly simple and cost-effective.

Casing 4 has a substantially rectangular shape and is made up of two sheets of said flexible plastic material welded along edge 4a. The weld is interrupted along the shorter sides to form a first 6a and a second 6b aperture. A vent tube 7 is inserted in the first aperture 7 comprising a hydrophobic filter 8. The hydrophobic filter 8 is for example a filter made of polyethylene.

The second aperture 6b instead serves for the passage of a dispensing tube 9 of the medical solution.

The dispensing tube 9 continues into casing 4 and is connected to a cannula 10. Cannula 10 comprises a connecting portion 10a with the dispensing tube 9 and an engagement portion 10b with the elastomeric pump 5, wherein said engagement portion 10b comprises, in distal position, a terminal aperture 10c.

The engagement portion 10b comprises, in position proximal to the connection portion 10a, a relief 25 that protrudes externally and preferably is an annular relief. Relief 25 comprises an outer profile 25a connected with the surface of the engagement portion 10b by means of a first inclined profile 25b and a second square profile 25c, wherein the first profile 25b is facing the terminal aperture 10c of the engagement portion 10b and the second profile 25c is facing the connection portion 10a.

The connection portion 10a has a larger diameter than the engagement portion 10b and is connected with the latter by means of a shoulder 26.

The elastomeric pump 5 consists of a balloon made of a thermoplastic elastomer.

The elastomeric pump is made of a polystyrene block copolymer hydrogenated with polyolefins (HSBS). Preferably, said polymers have a Shore A hardness of about 35 and a Shore D hardness of about 55.

It has been seen that such material, in addition to having a high elasticity and shape memory, has the property of not increasing the diameter over a certain inner pressure of the fluid, but of increasing the longitudinal extension thereof. This property is very important because it allows exerting a predetermined and constant hydrostatic pressure on the fluid over a wide filling range of the balloon.

The elastomeric pump 5 comprises a first portion 5a of engagement with the cannula 10, a second intermediate portion 5b and a third bottom portion 5c, wherein the second intermediate portion 5b has a convex outer profile. The elastomeric pump 5 comprises a longitudinal cavity 27 that comprises a first section 27a open at the end of the first portion 5a of pump 5 and a second section 27b, wherein the first section 27a has a larger diameter than the second section 27b and both sections 27a, 27b have a substantially cylindrical shape. The first section 27a of the longitudinal cavity 27 extends along the first 5a and part of the second portion 5b of the elastomeric pump 5, while the second section 27b extends to the bottom of the longitudinal cavity 27 and is connected to the first section 27a by means of a shoulder 28.

The first section 27a of the longitudinal cavity 27 comprises a recess 29 of complementary shape to relief 25 of cannula 10 and intended to snap-engage with it. The first inclined profile 25b of relief 25 is designed to facilitate the introduction and thus the engagement of cannula 10 into and with the longitudinal cavity 27.

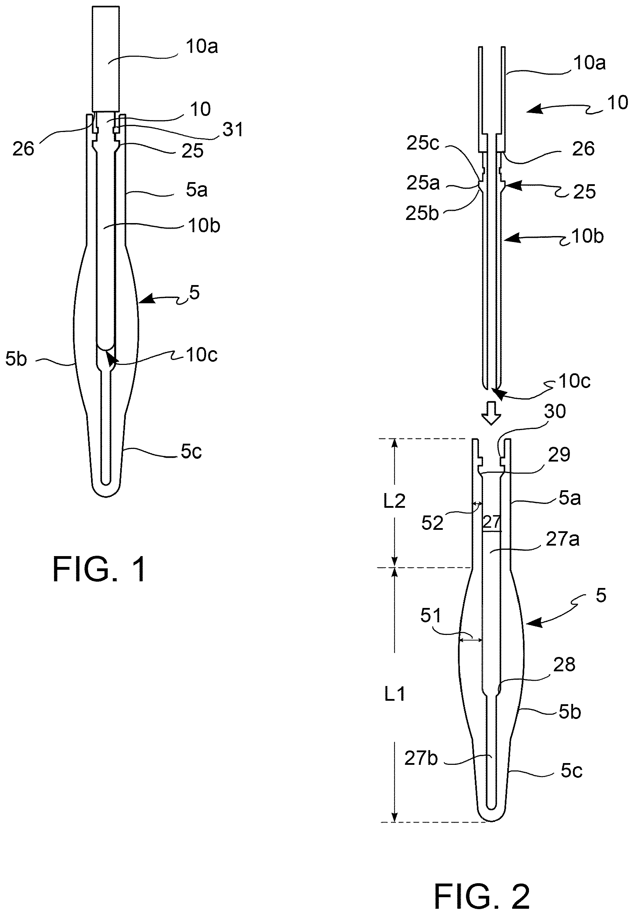

The first section 27a further comprises, in a position close to recess 29 and upstream of it along the direction of introduction of cannula 10, an annular relief 30. Between the annular relief 30 and the open end of the first section 27a, the inner diameter of the cavity is larger than the outer diameter of cannula 10 at that point, so as to form a spacing 31, as shown in figure 1.

It should be noted that, being both the first section 27a and the second section 27b of the longitudinal cavity 27 cylindrical, while the outer profile of the second intermediate portion 5b of the elastomeric pump 5 is convex, this second intermediate portion 5b has a wall thickness greater than that of the other two sections 5a, 5b and such a wall thickness is greatest at the point of greatest outer diameter of the second portion 5b of the elastomeric pump 5, decreasing towards the connecting point with both the first portion 5a and with the third portion 5c of the elastomeric pump 5. In this way, it was surprisingly seen that greater uniformity of the flow dispensed by the elastomeric pump is achieved, minimizing dispensing rate peaks which, in the prior art devices, usually occur at the initial and final dispensing steps.

In certain embodiments, the ratio between the maximum thickness S1 of the second portion 5b and the thickness S2 of the first portion 5a of the elastomeric pump 5 is between 1.2 and 3, or between 1.4 and 2.4, or between 1.5 and 2.35. In some embodiments, the maximum thickness S1 of the second portion 5b is between 2.4 and 5 mm, or between 2.7 and 4.95 mm or between 3.0 and 4.85 mm, while the thickness S2 of the first portion 5a of the elastomeric pump 5 is between 2 and 2.2 mm.

Another important parameter is the dimensional ratio between the sum L1 of the lengths of the second and third portion 5b, 5c of the elastomeric pump 5 and length L2 of the first portion 5a. In certain embodiments, this ratio is between 2.8 and 9 or between 2.9 and 6, or between 3 and 4. In certain embodiments, the length L1 is between 75 and 85 mm, or about 80 mm, while the length L1 is between 24 and 30 mm, or about 27 mm. In other embodiments, the length L2 is between 90 and 300 mm, or between 95 and 200 mm or between 100 and 150 mm, while the length L1 is between 30 and 38 mm, or about 34 mm.

In the embodiments described above, the diameter of the first section 27a of the longitudinal cavity 27 is preferably between 3.7 and 3.9 mm, while the diameter of the second section 27b is preferably between 1.9 and 2.1 mm.

The elastomeric pump 5 is sealably fixed to the surface of the engagement portion 10b of cannula 10 in a distal position with respect to the terminal aperture 10c, using for example a medical adhesive that can be cured by UV radiation, which is inserted in spacing 31 between cannula 10, the first section 27a of cavity 27 and the annular relief 30.

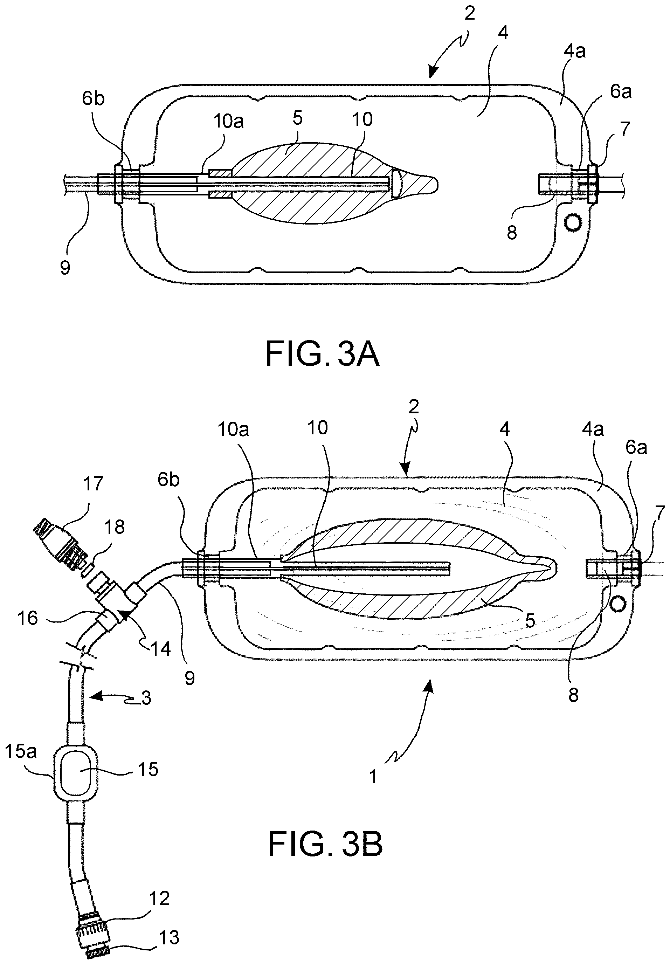

As shown in figures 3A and 3B, casing 4 has size both in length and width large enough compared to those of the elastomeric pump 5 filled with the solution to be dispensed, to create a substantially loose containment thereof. This prevents casing 4 from exerting pressure on the elastomeric pump 5 which would result in an extra undesired hydrostatic pressure on the medical solution to be dispensed. For example, both the length and the width of casing 4 will be twice those of the elastomeric pump 5.

The dispensing tube 9 ends, at the opposite end with respect to the elastomeric pump 5, with a connection element 12 with an infusion system such as a needle, a catheter or the like. For example, the connection element 12 may be a so-called male "luer-lock".

The connection element 12 comprises a hydrophobic filter 13, such as a polyethylene/polytetrafluoroethylene (PTFE) filter.

The dispensing tube 9 is further intercepted, along its path outside casing 4, by a valve means 14 for loading the medical solution and a filtering means 15 of the medical solution.

The valve means 14 comprises a valve 16, typically a non-return valve, to which a loading fitting 17 is connected through the interposition of a filtering means 18 of the medical solution. The loading fitting 17 for example is a "needle-less" valve. The filtering means 18 for example is a polyethylene/nylon filter.

The filtering means 15 of the medical solution is placed in an intermediate position between the valve means 14 and the connection element 12. The filtering means 15 is enclosed in a casing 15a laminar in shape with rectangular plan and is for example made of polycarbonate/polyethersulphone (PES) /PTFE.

The infusion device 1 according to the invention may be housed in a case 19.

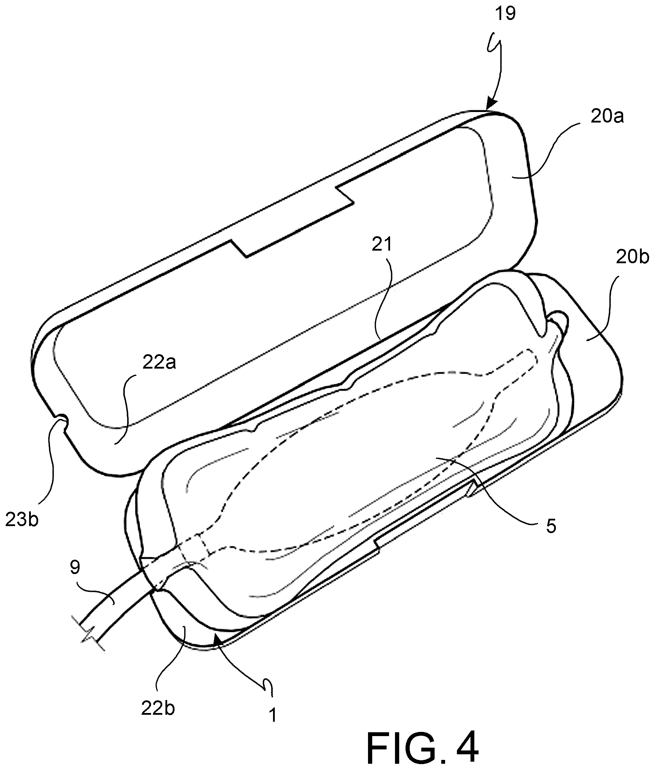

In certain embodiments, case 19 shown in figure 4, having a substantially parallelepiped shape, comprises two half-shells 20a, 20b joined along a hinge line 21. The two half-shells 20a, 20b comprise, along the respective joining edges 22a, 22b of a shorter side, recesses 23a, 23b which, joined when case 19 is closed, form an aperture having such a shape and size to let the dispensing tube 9 protrude.

The advantages of the infusion device 1 according to the invention are several and already partially discussed above.

First, the particular shape of the elastomeric pump allows an extremely constant dispensing of the medical solution, avoiding the onset of peaks in the initial and final stages of treatment, as is the case with the prior art devices.

Furthermore, the fact that casing 4 is not as rigid as in the prior art devices but flexible minimizes the dimensions and weight of the device and makes the manufacture, packaging and transport thereof easier and more cost-effective.

In addition, the elasto-mechanical features of the thermoplastic elastomer used to manufacture the elastomeric pump impart properties to the same such as to maximize the performance thereof.

It is clear that only some particular embodiments of the present invention have been described, and those skilled in the art will be able to make all the necessary modifications for its adaptation to particular applications, without departing from the protection scope of the present invention as defined in the accompanying claims.

## Claims

1. Infusion device (1) of a medical solution, comprising a pumping unit (2) connected to an infusion line (3), wherein the pumping unit (2) comprises a cannula (10) connected to said infusion line (3) and an elastomeric pump (5) connected to said cannula (10), **characterised in that** said elastomeric pump (5) comprises a first portion (5a) of engagement with the cannula (10), a second intermediate portion (5b) and a third bottom portion (5c), wherein the second intermediate portion (5b) has a convex-shaped outer profile and wherein the second intermediate portion (5b) has a wall thickness greater than the wall thickness of the first and third portion (5a, 5b) and such wall thickness is maximum at the point of greatest outside diameter of the second portion (5b) and slopes towards the connection point with both the first portion (5a) and the third portion (5c) of the elastomeric pump (5).

2. Infusion device (1) according to claim 1, wherein the cannula (10) comprises a connection portion (10a) with a dispensing tube (9) of the infusion line (3) and an engagement portion (10b) with the elastomeric pump (5), wherein said engagement portion (10b) comprises, in distal position, a terminal aperture (10c) and wherein the engagement portion (10b) comprises, in position proximal to the connection portion (10a), a relief (25) that protrudes externally.

3. Infusion device (1) according to claim 2, wherein said relief (25) comprises an outer profile (25a) connected with the surface of the engagement portion (10b) by means of a first inclined profile (25b) and a second square profile (25c), wherein the first profile (25b) is facing the terminal aperture (10c) of the engagement portion (10b) and the second profile (25c) is facing the connection portion (10a).

4. Infusion device (1) according to claim 2 or 3, wherein the connection portion (10a) has a larger diameter than the engagement portion (10b) and is connected with the latter by means of a shoulder (26).

5. Infusion device (1) according to any of claims 1 to 4, wherein the elastomeric pump (5) comprises a longitudinal cavity (27) that comprises a first section (27a) and a second section (27b), wherein the first section (27a) has a larger diameter than the second section (27b) and both sections (27a, 27b) have a substantially cylindrical shape.

6. Infusion device according to claim 5, wherein the first section (27a) of the longitudinal cavity (27) extends along the first (5a) and part of the second portion (5b) of the elastomeric pump (5), while the second section (27b) extends to the bottom of the longitudinal cavity (27).

7. Infusion device (1) according to claim 5 or 6, wherein the first section (27a) of the longitudinal cavity (27) comprises a recess (29) of complementary shape to the relief (25) of the cannula (10) and intended to snap-engage with it, and wherein, in position next to the recess (29) and upstream of it along the direction of introduction of the cannula (10), is disposed an annular relief (30) intended to go into abutment with the shoulder (26) of the cannula (10).

8. Infusion device (1) according to any of claims 1 to 7, wherein the ratio between the maximum thickness (S1) of the second portion (5b) and the thickness (S2) of the first portion (5a) of the elastomeric pump (5) is between 1.2 and 3, or between 1.4 and 2.4, or between 1.5 and 2.35.

9. Infusion device (1) according to any of claims 1 to 8, wherein the ratio between the sum (L1) of the lengths of the second and third portions (5b, 5c) of the elastomeric pump (5) and the length (L2) of the first portion (5a) is between 2.8 and 9, or between 2.9 and 6, or between 3 and 4.

10. Infusion device (1) according to any of claims 1 to 9, wherein the elastomeric pump (5) is constituted by a balloon made of a thermoplastic elastomer comprising a block copolymer of hydrogenated polystyrene and polyolefins.

11. Infusion device (1) according to claim 10, wherein said thermoplastic elastomer has a Shore A hardness of about 35 and a Shore D hardness of about 55.

12. Infusion device (1) according to any of claims 1 to 11, wherein the elastomeric pump (5) is contained in a casing (4) formed by two sheets of thermoplastic material welded along the edge (4a), the weld being interrupted along the shorter sides to form a first (6a) and a second aperture (6b), wherein in the first aperture (6a) is inserted a vent tube (7) that comprises a hydrophobic filter (8), preferably a polyethylene filter.

13. Infusion device (1) according to any of claims 2 to 12, wherein the dispensing tube (9) ends, at the opposite end with respect to the elastomeric pump (5), with a connection element (12) with an infusion system such as a needle, a catheter or the like.

14. Infusion device according to claim 13, wherein the connection element (12) comprises a hydrophobic filter (13), preferably a polyethylene/polytetrafluoroethylene (PTFE) filter.

15. Infusion device according to any of claims 2 to 14, wherein the dispensing tube (9) is intercepted by a valve means (14) for loading the medical solution and a filtering means (15) of the medical solution.

## Patentansprüche

1. Infusionsvorrichtung (1) einer medizinischen Lösung, die eine mit einer Infusionsleitung (3) verbundene Pumpeinheit (2) aufweist, wobei die Pumpeinheit (2) eine mit der Infusionsleitung (3) verbundene Kanüle (10) und eine mit der Kanüle (10) verbundene Elastomerpumpe (5) aufweist, **dadurch gekennzeichnet, dass** die Elastomerpumpe (5) einen ersten Abschnitt (5a) einer Ineingriffnahme mit der Kanüle (10), einen zweiten, dazwischen liegenden Abschnitt (5b) und einen dritten, unteren Abschnitt (5c) aufweist, wobei der zweite, dazwischen liegende Abschnitt (5b) ein konvexes Außenprofil aufweist und wobei der zweite, dazwischen liegende Abschnitt (5b) eine größere Wanddicke aufweist als der erste und der dritte Abschnitt (5a, 5b), und derart, dass die Wanddicke an dem Punkt des größten Außendurchmessers des zweiten Abschnitts (5b) am größten ist und zu dem Verbindungspunkt sowohl mit dem ersten Abschnitt (5a) als auch mit dem dritten Abschnitt (5c) der Elastomerpumpe (5) hin abfällt.

2. Infusionsvorrichtung (1) gemäß Anspruch 1, bei der die Kanüle (10) einen Verbindungsabschnitt (10a) mit einem Abgabeschlauch (9) der Infusionsleitung (3) und einen Ineingriffnahmeabschnitt (10b) mit der Elastomerpumpe (5) aufweist, wobei der Ineingriffnahmeabschnitt (10b) in distaler Position eine Abschlussöffnung (10c) aufweist und wobei der Ineingriffnahmeabschnitt (10b) in proximaler Position zu dem Verbindungsabschnitt (10a) einen Absatz (25) aufweist, der nach außen vorsteht.

3. Infusionsvorrichtung (1) gemäß Anspruch 2, bei der der Absatz (25) ein Außenprofil (25a) aufweist, das mittels eines ersten geneigten Profils (25b) und eines zweiten quadratischen Profils (25c) mit der Oberfläche des Ineingriffnahmeabschnitts (10b) verbunden ist, wobei das erste Profil (25b) der Abschlussöffnung (10c) des Ineingriffnahmeabschnitts (10b) zugewandt ist und das zweite Profil (25c) dem Verbindungsabschnitt (10a) zugewandt ist.

4. Infusionsvorrichtung (1) gemäß Anspruch 2 oder 3, bei der der Verbindungsabschnitt (10a) einen größeren Durchmesser aufweist als der Ineingriffnahmeabschnitt (10b) und anhand eines Vorsprungs (26) mit Letzteren verbunden ist.

5. Infusionsvorrichtung (1) gemäß einem der Ansprüche 1 bis 4, bei der die Elastomerpumpe (5) einen länglichen Hohlraum (27) aufweist, der ein erstes Segment (27a) und eine zweites Segment (27b) aufweist, wobei das erste Segment (27a) einen größeren Durchmesser aufweist als das zweite Segment (27b) und beide Segmente (27a, 27b) eine im Wesentlichen zylindrische Form aufweisen.

6. Infusionsvorrichtung gemäß Anspruch 5, bei der sich das erste Segment (27a) des länglichen Hohlraums (27) entlang des ersten (5a) und eines Teils des zweiten Abschnitts (5b) der Elastomerpumpe (5) erstreckt, während sich das zweite Segment (27b) bis zu dem unteren Ende des länglichen Hohlraums (27) erstreckt.

7. Infusionsvorrichtung (1) gemäß Anspruch 5 oder 6, bei der das erste Segment (27a) des länglichen Hohlraums (27) eine Aussparung (29) einer zu dem Absatz (25) der Kanüle (10) komplementären Form aufweist, die mit dem Absatz (25) auf einrastende Weise in Eingriff gelangen soll, und bei der in einer Position neben der Aussparung (29) und in Strömungsrichtung vor derselben entlang der Richtung der Einführung der Kanüle (10) ein ringförmiger Absatz (30) angeordnet ist, der mit dem Vorsprung (26) der Kanüle (10) zur Anlage gelangen soll.

8. Infusionsvorrichtung (1) gemäß einem der Ansprüche 1 bis 7, bei der das Verhältnis zwischen der maximalen Dicke (S1) des zweiten Abschnitts (5b) und der Dicke (S2) des ersten Abschnitts (5a) der Elastomerpumpe (5) zwischen 1,2 und 3 oder zwischen 1,4 und 2,4 oder zwischen 1,5 und 2,35 liegt.

9. Infusionsvorrichtung (1) gemäß einem der Ansprüche 1 bis 8, bei der das Verhältnis zwischen der Summe (L1) der jeweiligen Länge des zweiten und des dritten Abschnitts (5b, 5c) der Elastomerpumpe (5) und der Länge (L2) des ersten Abschnitts (5a) zwischen 2,8 und 9 oder zwischen 2,9 und 6 oder zwischen 3 und 4 beträgt.

10. Infusionsvorrichtung (1) gemäß einem der Ansprüche 1 bis 9, bei der die Elastomerpumpe (5) durch einen Ballon gebildet ist, der aus einem thermoplastischen Elastomer hergestellt ist, das ein Blockcopolymer aus hydriertem Polystyrol und Polyolefinen aufweist.

11. Infusionsvorrichtung (1) gemäß Anspruch 10, bei der das thermoplastische Elastomer eine Shore-A-Härte von etwa 35 und eine Shore-D-Härte von etwa 55 aufweist.

12. Infusionsvorrichtung (1) gemäß einem der Ansprüche 1 bis 11, bei der die Elastomerpumpe (5) in einem Gehäuse (4) enthalten ist, das durch zwei Lagen eines thermoplastischen Materials gebildet ist, die am Rand (4a) entlang angeschweißt sind, wobei die Schweißung entlang der kürzeren Seiten unterbrochen ist, um eine erste (6a) und eine zweite Öffnung (6b) zu bilden, wobei in die erste Öffnung (6a) ein Entlüftungsschlauch (7) eingefügt ist, der ein hydrophobes Filter (8), vorzugsweise ein Polyethylenfilter, aufweist.

13. Infusionsvorrichtung (1) gemäß einem der Ansprüche 2 bis 12, bei der der Abgabeschlauch (9) an dem bezüglich der Elastomerpumpe (5) gegenüberliegenden Ende mit einem Verbindungselement (12) mit einem Infusionssystem wie z. B. einer Nadel, einem Katheter oder dergleichen endet.

14. Infusionsvorrichtung gemäß Anspruch 13, bei der das Verbindungselement (12) ein hydrophobes Filter (13), vorzugsweise ein Polyethylen/Polytetrafluorethylen(PTFE)-Filter, aufweist.

15. Infusionsvorrichtung gemäß einem der Ansprüche 2 bis 14, bei der der Abgabeschlauch (9) durch eine Ventileinrichtung (14) zum Beschicken der medizinischen Lösung und durch eine Filtereinrichtung (15) der medizinischen Lösung unterteilt ist.

## Revendications

1. Dispositif de perfusion (1) pour solution médicale, comprenant une unité de pompage (2) raccordée à une ligne de perfusion (3), dans lequel l'unité de pompage (2) comprend une canule (10) raccordée à ladite ligne de perfusion (3) et une pompe élastomère (5) raccordée à ladite canule (10), **caractérisé en ce que** ladite pompe élastomère (5) comprend une première partie d'insertion (5a) dans la canule (10), une deuxième partie intermédiaire (5b) et une troisième partie inférieure (5c), dans lequel la deuxième partie intermédiaire (5b) a un profil extérieur de forme convexe et dans lequel la deuxième partie intermédiaire a une épaisseur de paroi supérieure à l'épaisseur de paroi des première et troisième parties (5a, 5c) et cette épaisseur de paroi est maximale au point de plus grand diamètre extérieur de la deuxième partie (5b) et diminue en direction du point de jonction avec à la fois la première partie (5a) et troisième partie (5c) de la pompe élastomère (5).

2. Dispositif de perfusion (1) selon la revendication 1, dans lequel la canule (10) comprend une partie de raccordement (10a) à un tube de distribution (9) de la ligne de perfusion (3) et une partie d'insertion (10b) dans la pompe élastomère (5), dans lequel ladite partie d'insertion (10b) comprend, en position distale, une ouverture terminale (10c) et dans lequel ladite partie d'insertion (10b) comprend, en position proximale par rapport à la partie de raccordement (10a), un relief (25) qui fait saillie vers l'extérieur.

3. Dispositif de perfusion (1) selon la revendication 2, dans lequel ledit relief (25) a un profil extérieur (25a) joint à la surface de la partie d'insertion (10b) au moyen d'un premier profil incliné (25b) et d'un second profil carré (25c), dans lequel le premier profil (25b) est en regard de l'ouverture terminale (10c) de la partie d'insertion (10b) et le second profil (25c) en regard de la partie de raccordement (10a).

4. Dispositif de perfusion (1) selon la revendication 2 ou 3, dans lequel la partie de raccordement (10a) a un diamètre supérieur à la partie d'insertion (10b) et est jointe à cette dernière au moyen d'un épaulement (26).

5. Dispositif de perfusion (1) selon l'une quelconque des revendications 1 à 4, dans lequel la pompe élastomère (5) comporte une cavité longitudinale (27) qui comprend une première section (27a) et une seconde section (27b), dans lequel la première section (2a) a un diamètre supérieur à la seconde section (27b) et les deux sections (27a, 27b) ont une forme sensiblement cylindrique.

6. Dispositif de perfusion selon la revendication 5, dans lequel la première section (27a) de la cavité longitudinale (27) s'étend le long de la première partie (5a) et partiellement de la deuxième partie (5b) de la pompe élastomère (5), tandis que la seconde section (27b) s'étend vers le fond de la cavité longitudinale (27).

7. Dispositif de perfusion (1) selon la revendication 5 ou 6, dans lequel la première section (27a) de la cavité longitudinale (27) comporte un évidement (29) de forme complémentaire au relief (25) de la canule (10) et destiné à s'encliqueter avec celui-ci et dans lequel, en une position proche de l'évidement (29) et en amont de celui-ci dans le sens d'introduction de la canule (10) est disposé un relief annulaire (30) destiné à venir en butée contre l'épaulement (26) de la canule (10).

8. Dispositif de perfusion (1) selon l'une quelconque des revendications 1 à 7, dans lequel le rapport entre l'épaisseur maximale (S1) de la deuxième partie (5b) et l'épaisseur (S2) de la première partie (5a) de la pompe élastomère (5) est compris entre 1,2 et 3, ou entre 1,4 et 2,4, ou entre 1,5 et 2,35.

9. Dispositif de perfusion (1) selon l'une quelconque des revendications 1 à 8, dans lequel le rapport entre la somme (L1) des longueurs des deuxième et troisième parties (5b, 5c) de la pompe élastomère (5) et la longueur (L2) de la première partie (5a) est compris entre 2,8 et 9, ou entre 2,9 et 6, ou entre 3 et 4.

10. Dispositif de perfusion (1) selon l'une quelconque des revendications 1 à 9, dans lequel la pompe élastomère (5) est constituée d'un ballon en élastomère thermoplastique comprenant un copolymère en bloc de polystyrène hydrogéné et polyoléfines.

11. Dispositif de perfusion (1) selon la revendication 10, dans lequel ledit élastomère thermoplastique a une dureté Shore A d'environ 35 et une dureté Shore D d'environ 55.

12. Dispositif de perfusion (1) selon l'une quelconque des revendications 1 à 11, dans lequel la pompe élastomère (5) est logée dans un boîtier (4) formé par deux feuilles de matériau thermoplastique soudées sur le bord (4a), la soudure étant interrompue le long des petits côtés pour former une première (6a) et une seconde ouverture (6b), dans lequel dans la première ouverture (6a) est inséré un tube d'aération (7) qui comprend un filtre hydrophobe (8), de préférence un filtre en polyéthylène.

13. Dispositif de perfusion (1) selon l'une quelconque des revendications 2 à 12, dans lequel le tube de distribution (9) se termine, à l'extrémité opposée par rapport à la pompe élastomère (5), par un élément de raccordement (12) à un système de perfusion tel qu'une aiguille, un cathéter ou autre.

14. Dispositif de perfusion selon la revendication 13, dans lequel l'élément de raccordement (12) comporte un filtre hydrophobe (13), de préférence un filtre en polyéthylène/ polytétrafluoroéthylène (PTFE).

15. Dispositif de perfusion selon l'une quelconque des revendications 2 à 14, dans lequel le tube de distribution (9) est intercepté par un moyen de vanne (14) pour charger la solution médicale et par un moyen de filtration (15) de la solution médicale.
